Europäisches Patentamt

**European Patent Office**

Office européen des brevets

(11) Publication number: **0 208 362**
**A1**

(12) # EUROPEAN PATENT APPLICATION

(21) Application number: **86201053.5**

(22) Date of filing: **18.06.86**

(51) Int. Cl.⁴: **A 61 K 33/06**
**A 61 K 33/26, A 61 K 9/52**
**A 61 K 9/24**

(30) Priority: **28.06.85 US 750566**

(43) Date of publication of application:
**14.01.87 Bulletin 87/3**

(84) Designated Contracting States:
**BE CH DE FR GB IT LI LU NL**

(71) Applicant: **THE PROCTER & GAMBLE COMPANY**
**One Procter & Gamble Plaza**
**Cincinnati Ohio 45202(US)**

(72) Inventor: **Anastasia, Frank Bernard**
**7020 Kirkcaldy Drive**
**West Chester Ohio 45069(US)**

(72) Inventor: **Smith, Kenneth Thomas**
**7013 Noble Avenue**
**Cincinnati Ohio 45239(US)**

(72) Inventor: **Kelm, Gary Robert**
**8524 Althaus Road**
**Cincinnati Ohio 45247(US)**

(72) Inventor: **Boggs, Robert Wayne**
**725 Greenville Avenue**
**Cincinnati Ohio 45246(US)**

(74) Representative: **Ernst, Hubert et al,**
**PROCTER & GAMBLE EUROPEAN TECHNICAL CENTER**
**Temselaan 100**
**B-1820 Strombeek-Bever(BE)**

(54) Dietary supplements containing iron and enterically coated calcium.

(57) A dietary calcium and iron supplement is disclosed. This supplement comprises nutritional amounts of both calcium and iron. The supplement also includes an enteric or like delayed release coating which surrounds the calcium. The enteric coating substantially prevents the calcium from inhibiting the biological absorption of the iron. As a result, calcium and iron can be simultaneously without adverse mineral interactions.

EP 0 208 362 A1

# DIETARY SUPPLEMENTS CONTAINING IRON AND ENTERICALLY COATED CALCIUM

Frank B. Anastasia

Gary R. Kelm

Kenneth T. Smith

Robert W. Boggs

## TECHNICAL FIELD

This application relates to dietary supplements useful in simultaneously delivering iron and calcium.

Calcium and iron are important minerals in the human diet. Calcium is the primary mineral in bone building. Iron is a key metal for a number of biologically important compounds, especially hemoglobin in blood. Chronic calcium-deficiency can eventually result in the serious bone loss disease known as osteoporosis. Failure to include enough iron in the diet can lead to a number of disorders, in particular iron-deficiency anemia.

Those individuals who are at significant risk to calcium or iron deficiencies need to increase their dietary intake of these minerals. This can be accomplished by including in the diet foods rich in calcium and iron. However, compliance with such diets is often difficult, especially in today's world of eating on the go, ready-to-serve meals and diet fads. Frequently, supplement tablets containing these minerals are used to insure an adequate dietary intake of calcium and iron.

Certain population groups, in particular pre-menopausal women, are prone to both calcium and iron deficiencies. However, taking calcium and iron at the same time can lead to adverse interactions between these minerals. It is known that calcium supplementation can adversely affect iron absorption in animals. See Barton et al., "Calcium Inhibition of Inorganic Iron Absorption in Rats," Gastroenterology, Vol. 84, (1983), pp. 90-101 (rats consuming high calcium diets of marginal iron content developed mild iron-deficiency anemia); Chapman et al., "Effect of Calcium and Phosphorous Salts on the Utilization of Iron by Anemic Rats," Brit. J. Nutr., Vol. 11, (1957), pp. 127-33 (addition of relatively large amounts of calcium salts to bread diets adversely affects utilization of iron by anemic rats). It has also been found in clinical studies involving women conducted for The Procter & Gamble Company (the assignee of this application), that simultaneous administration of calcium and iron decreases the absorption of iron.

The U.S. recommended daily allowance (USRDA) of calcium is 1000 mg/day. By contrast, the USRDA for iron is 18 mg/day. Accordingly, a high calcium to iron weight ratio is required to meet human nutritional needs. However, it is believed that a high calcium to iron ratio causes the adverse interaction between these minerals. By being in large excess, calcium interferes with iron absorption in the small intestine. The result is a substantial decrease in the amount of iron absorbed.

There are several ways to avoid this calcium-iron interaction. One is the time separated dose method where calcium and iron supplements are taken at different times. It has been found that taking a calcium supplement at least two hours after the iron supplement avoids adverse mineral interactions. However, this time separated dose method is burdensome because the individual must remember when to take the calcium supplement. This burden can be especially great since the time between taking the iron and calcium supplements is relatively long (two hours).

Another method is to minimize the level of calcium in the supplement. See U.S. Patent 4,431,634 to Ellenbogen, filed December 21, 1981, issued February 14, 1984 (bioavailability of iron enhanced by minimizing the level of calcium and magnesium carbonates and oxides to no more than 300 mg and 75 mg, respectively). This method appears to operate by reducing the amount of calcium which can interfere with iron absorption in the intestine. However, minimizing the level of calcium in the supplement would require multiple, time-spaced doses to achieve the rather high USRDA requirements for calcium. Accordingly, it would be highly desirable to provide a supplement which can

simultaneously deliver iron and high levels of calcium without adverse mineral interactions.

## BACKGROUND ART

### A.   Calcium Interactions with Iron

Barton et al., "Calcium Inhibition of Inorganic Iron Absorption in Rats," Gastroenterology, Vol. 84, (1983), pp. 90-101, describe a study on the relationship between dietary calcium content and iron absorption in experiments involving rats.  In these experiments, the rats were fed diets containing low to high levels of calcium in conjunction with normal to severely low levels of iron. Animals fed the high calcium, normal iron diet developed iron depletion while rats consuming the high calcium diets of marginal iron content developed mild iron deficiency anemia.  From this, Barton et al. concluded that calcium significantly diminished the absorption of ferrous and ferric iron in a dose-related manner. Barton et al. further suggest that individuals consuming a high calcium diet containing marginal amounts of iron could develop iron deficiency anemia and that this may explain why infants fed cow's milk have a greater incidence of iron deficiency anemia than those fed human milk.  (Cow's milk has 3 to 4 times the concentration of calcium compared to human milk).  See also Chapman et al., "Effect of Calcium and Phosphorous Salts on the Utilization of Iron by Anaemic Rats," Brit. J. Nutr., Vol. II, (1957), pp. 127-33 (addition of relatively larger amounts of calcium salts to bread diets adversely affects utilization of iron by anemic rat).

Monsen et al., "Food Iron Absorption in Human Subjects:  The Effects of Calcium and Phosphate Salts on the Absorption of Nonheme Iron," Am. J. Clin. Nutr., Vol. 29, (1976), pp. 1142-48, describes a clinical study where test meals containing varying levels of calcium and phosphorous were fed to 34 male and female volunteers ranging from 17-34 years in age.  From these studies, Monsen et al. concluded that a significant reduction in nonheme iron absorption was observed in the presence of calcium and phosphate salts as compared to absorption in their absence.

- 4 -

0208362

U.S. Patent 4,431,634 to Ellenbogen, filed December 21, 1981, issued February 14, 1984, discloses a prenatal multi-mineral supplement composition having enhanced iron bioavailability. The bioavailability of iron is enhanced by minimizing the level of calcium and magnesium carbonates and oxides present to no more than 300 mg and 75 mg, respectively. Indeed, the level of calcium and magnesium in these supplements can be reduced to 0. See column 2, line 20-22. See also, Seligman et al., "Measurements of Iron Absorption from Prenatal Multivitamin Supplements," Obstetrics & Gyn., Vol. 61, (1983), pp. 356-62, where the adverse effect of calcium carbonate and magnesium oxide on the absorption of prenatal iron supplements is also noted.

B.    Separating components in calcium supplement

U.S. Patent 3,345,265 to Grodberg et al., issued October 3, 1967, discloses a multi-layer vitamin and mineral tablet containing sodium fluoride and calcium carbonate. As described in this patent, sodium fluoride reacts with calcium carbonate to form undesired, insoluble calcium fluoride. This problem is solved by physically separating the sodium fluoride and calcium carbonate in the tablet. Physical separation is accomplished by forming calcium carbonate, vitamins and other minerals (e.g. ferrous sulfate) into a core which is separated from the sodium fluoride by at least one layer of a soluble sugar coating. By the time the calcium carbonate goes into solution, the sodium fluoride from the tablet has been dissolved and absorbed.

U.S. Patent 2,410,417 to Anderson, issued November 5, 1946, discloses a vitamin and mineral supplement which contains calcium, preferably as the phosphate salt. The vitamins, as well as certain of the minerals, are separated or isolated through the use of protective coatings to prevent adverse interactions. In particular, iron is isolated from the vitamins through the use of a protective coating. See also U.S. Patent 2,887,436 to Klioze et al., issued May 19, 1959, which discloses dietary supplements containing calcium and iron where the incompatible vitamins and minerals are incorporated into separate granules.

**C.** Enteric coatings for aspirin

It is well known that analgesics such as aspirin cause irritation of the stomach. To prevent this irritation, the aspirin particles can be enterically coated to permit passage through the stomach and into the intestines where the coating is dissolved so that the aspirin is released and absorbed. Representative of such enterically coated aspirin products are those disclosed in European Patent Application 94,123 to Dahl, published November 16, 1983; European Patent Application 94,116 to Close et al., published November 16, 1983; and European Patent Application 94,117 to Leis et al., published November 16, 1983.

## DISCLOSURE OF THE INVENTION

The present invention relates to dietary calcium and iron supplements. The supplement comprises nutritionally effective amounts of both a source of absorbable and bioavailable calcium and source of absorbable and bioavailable iron. The supplement further includes a delayed release coating which surrounds the calcium source. The iron source is positioned in the supplement so that the delayed release coating substantially prevents the calcium source from inhibiting the biological absorption of iron in the iron source.

The dietary calcium and iron supplements of the present invention permit the simultaneous delivery of these minerals (particularly a desirable high weight ratio of calcium to iron) without adverse interactions. It is believed that the delayed release coating surrounding the calcium source substantially prevents the release of this mineral until after the supplement reaches the small intestine. It is also believed that the iron in the supplement is substantially absorbed prior to the release of the calcium. Accordingly, the mechanism which causes interaction between these two minerals is avoided.

**A.** General Definitions

As used herein, the term "pharmaceutically acceptable" or "pharmacologically acceptable" means the ingredients used in the supplements of the present invention are suitable for use by humans or other mammals, without undue toxicity, irritation,

allergic response, or the like, commensurate with a reasonable benefit/risk ratio.

As used herein, the term "comprising" means various other compatible components, including both active and inert ingredients, can be conjointly employed in the supplements of the present invention. The term "comprising" encompasses the more restrictive terms "consisting essentially of" and "consisting of".

B.   Dietary Calcium and Iron Supplement

The dietary supplement of the present invention basically comprises: (1) a source of calcium; (2) a delayed release coating; and (3) a source of iron.

Suitable sources of calcium are those calcium salts which are pharmaceutically acceptable, biologically absorbable and provide bioavailable calcium. Generally, preferred calcium salts are those which are the most absorbable and bioavailable and which have the densest crystalline forms. Suitable calcium salts include calcium carbonate, calcium bicarbonate, calcium citrate, calcium malate, calcium phosphate, calcium oxide, calcium hydrogen phosphate, calcium dihydrogen phosphate, calcium lactate, calcium orotate, calcium chloride, calcium hydroxide, calcium sulfate, calcium gluconate, calcium levulinate, calcium hydroxyapatite, calcium caseinate, calcium glubionate, and mixtures thereof. These calcium salts can be obtained synthetically, from mineral sources or from animal sources, e.g. oyster shells, bone meal, egg shells.

Particularly preferred calcium salts are calcium carbonate, and mixtures of calcium citrate and calcium malate. With regard to calcium carbonate, the crystalline forms known as calcite, or the more dense form known as aragonite, can be used. With regard to mixtures of calcium citrate and calcium malate, citric and malic acid (1:1 mole ratio) are dissolved in water and then calcium carbonate is added to this solution such that the mole ratio of calcium carbonate to citric acid to malic acid is from about 1.0:2.0:2.0 to about 1.0:0.1:0.1, preferably 1.0:0.5:0.5. After the evolution of carbon dioxide has ceased, the solution is dried

(freeze dried or oven dried) to obtain the mixture of calcium citrate and calcium malate.

The calcium source is included in the supplement in a nutritionally effective amount. As used herein, the term "nutritionally effective amount" with regard to the calcium source refers to an amount of calcium sufficient to provide a nutritional benefit to the individual taking the supplement. The particular amount of calcium included in the supplement typically depends upon the calcium source used and its density (usually determined by its crystalline form), as well as the nutritional benefits desired. Usually, the calcium source is included in the supplement at from about 100 to about 1500 mg. based on the weight of the calcium present in the source. Preferably, the calcium source is included in an amount from about 100 to about 1000 mg. by weight calcium. The amount of calcium included in the supplement can be a portion or all of the U.S. recommended daily allowance (USRDA) for calcium.

A key component in the supplements of the present invention is the delayed release coating. As used herein, the term "delayed release coating" refers to a pharmaceutically acceptable coating which at least substantially prevents the release of the calcium source for absorption until it reaches the small intestine. As used herein, "substantially prevents" typically means that greater than about 90% of the calcium source has not been dissolved by the time the supplement reaches the small intestine. (It is to be understood that the term "substantially prevents" can include situations where more than 10% of the calcium source is dissolved, so long as the undesirable interactions between calcium and iron are avoided.) These delayed release coatings generally fall into four categories: (1) pH-sensitive; (2) enzyme-sensitive; (3) infusive; and (4) slowly soluble.

The pH-sensitive coatings are referred to in the present application as enteric coatings. As used herein, the term "enteric coating" includes any pharmaceutically acceptable coating insoluble at pHs of from about 1 to about 3 while being soluble at pHs in the range of from about 4.5 to about 8.0. Suitable

enteric materials include hydroxypropyl methylcellulose phthalate, cellulose acetate phthalate, cellulose acetate succinate, acrylic and methacrylic anionic polymers such as Eudragit L and S sold by Rohm Pharma, and polyvinyl acetyl phthalate, as well as mixtures of these materials. The particularly preferred enteric material is cellulose acetate phthalate. Optional components suitable for inclusion in the enteric coating are surface active agents, inert solids such as talc, and plasticizers such as triethyl citrate, triacetin, triglycerides and acetylated monoglycerides.

The enzyme-sensitive coatings are referred to in the present application as enteral enzyme degradable coating. As used herein, the term "enteral enzyme degradable coating" refers to any pharmaceutically acceptable coating which remains sub-stantially intact in the stomach but which is degradable by enzymes in the small intestine. Suitable enteral enzyme degradable materials include the lipids, such as naturally occurring fat and oils, as well as protein materials such as zein and albumin. Other suitable lipids include glyceryl tristearate, glyceryl tripalmitate, monopalmityl distearyl triglyceride, dipalmityl monostearyl trigylceride, sorbitan tristearate, hexaglycerol hexapalmitate, hexaglycerol hexastearate, decaglycerol decapalmitate, decaglycerol decastearate, and mixtures thereof. As with the enteric coatings, optional components such as plasticizers and binders can be included in the enzyme degradable coatings.

The next category of delayed release coatings are the infusive ones. As used herein, the term "infusion coating" refers to any pharmaceutically acceptable coating which remains intact in the gastrointestinal tract, but permits the infusion of digestive fluids to dissolve the calcium source. Suitable infusive coating materials include ethyl cellulose, cellulose acetate, and quaternary ammonium substituted acrylic resins such as Eudragit RL and RS sold by Röhm Pharma. As with the enteric and enzyme degradable coatings, optional components such as plasticizers and soluble materials to enhance diffusion can be included in the infusive coatings.

The last category of delayed release coatings are the slowly soluble type. As used herein, the term "slowly soluble coating" refers to any pharmaceutically acceptable coating whose dissolution rate in digestive fluids is such that the calcium source is not substantially released until the supplement reaches the small intestine. The slowly soluble coating composition and thickness are usually adjusted so that the calcium source is released about 2 hours or more after ingestion of the supplement. Suitable materials for slowly soluble coatings include the polyethylene glycols, glycerol monostearate and beeswax.

The delayed release coating material is used in an amount sufficient to provide an adequate coating to surround the calcium source to prevent it from inhibiting the biological absorption of the iron in the iron source. The amount of delayed release material necessary to provide an adequate coating is highly dependent on the coating used and the surface area of the calcium source which needs to be surrounded. (The surface area of the calcium source is highly dependent on the amount used as well as its form.) For tablet products to be hereafter defined, the amount of delayed release coating material necessary is typically from about 1 to about 20% by weight of the calcium source and preferably from about 2 to about 10% by weight of the calcium source. For capsule products to be hereafter defined, the amount of delayed release coating material necessary is typically from about 2 to about 25% by weight of the calcium source, and preferably from about 5 to about 15% by weight of the calcium source.

Iron sources which can be used in the supplement of the present invention are those which are pharmaceutically acceptable, biologically absorbable and provide bioavailable iron. Accordingly, both heme and nonheme iron sources can be used. Generally, suitable iron sources are those iron salts which are water-soluble, especially the ferrous salts. Suitable iron salts include ferrous sulfate, ferrous chloride, ferrous fumarate, ferrous gluconate, ferrous ascorbate, ferrous succinate, ferrous lactate, ferric ammonium citrate, ferric pyrophosphate, ferroglycine sulfate, and mixtures thereof. The preferred iron salts are ferrous sulfate and ferrous fumarate.

The iron source is included in the supplement in a nutritionally effective amount. As used herein, the term "nutritionally effective amount" with regard to the iron source refers to an amount of iron sufficient to provide nutritional benefits to the individual taking the supplement. The amount of iron included in the supplement will typically depend upon the nutritional needs of the individual. Suitable amounts of the iron source range from about 1 to about 50 mg. based on the weight of iron present in the iron source. Preferred levels of the iron source range from about 6 to about 25 mg. by weight iron. The amount of iron included in the supplement can be a portion or all of the USRDA for iron.

Within the weight ranges of calcium and iron specified, the weight ratio of calcium relative to iron is especially important. As previously discussed, it is believed that the reason for the adverse interaction between these minerals is due to the large amount of dietary calcium required relative to the small amount of dietary iron. Accordingly, the supplements of the present invention are particularly suitable for simultaneously delivering this high weight ratio of calcium to iron. In terms of defining these weight ratios, the amount of calcium in the calcium source is measured relative to the amount of iron in the iron source. Typically, the weight ratio of calcium to iron is from about 10:1 to about 150:1. Preferably, the weight ratio of calcium to iron is from about 20:1 to about 90:1.

The iron source is positioned in the supplement so that the delayed release coating can substantially prevent the calcium source from inhibiting the biological absorption of iron from the iron source. This usually means placing the iron source so that it is out of contact with the calcium source. For example, the iron source can be formulated as a discrete component from the coated calcium source. Typically, the iron source is adhered to the exterior surface of the delayed release coating.

Other optional ingredients typically included in dietary supplements or other pharmaceutical compositions can be used. For example, vitamins and other minerals can be used in dietary supplements of the present invention. Suitable vitamins include A, D, E, C, $B_1$, $B_2$, $B_6$, $B_{12}$, niacin, folic acid, thiamine, biotin and riboflavin. Other minerals which can be included in the supplement include zinc, potassium, magnesium, manganese and copper. Those vitamins and minerals which adversely interact with calcium should be positioned in the supplement so that the delayed release coating can substantially prevent the calcium source from interacting with them.

B. Method for making dietary supplements in tablet and capsule form

The dietary calcium and iron supplements of the present invention can be made in capsule or tablet form. See Lieberman et al, Pharmaceutical Dosage Forms: Tablets, Volume 3, (1982), pp. 108-110 (incorporated by reference), for a general description of methods for making tablets with delayed release coatings.

For the capsule form, the supplement comprises a suitable calcium source in the form of a multiplicity of granules. These granules typically have a particle size of about 250 microns to about 2000 microns. Preferably, these granules have a particle size of from about 500 to about 1200 microns. The coating of delayed release material surrounds each of the granules. A suitable iron source is included as either discrete granules or is preferably adhered to the exterior surface of the delayed release coating of each of the calcium granules. The granules are contained within an edible casing typically made from gelatin or other suitable edible material.

In making the capsule form of the supplement, the calcium source can be formed into granules by several methods. Examples of these methods include coating of inert substrates using fluidized beds, CF granulators or similar devices, roller compaction or slugging and then milling, direct spheronization using fluidized beds, and extrusion/spheronization. The calcium granules formed by these methods are sieved to the desired particle size. The calcium granules are then coated with the delayed release material from a solvent solution, a latex suspension or a hot melt. The iron source is then adhered to each of the coated calcium granules using a fluidized bed, CF granulator or similar device. Alternatively, separate granules of the iron source can be prepared as above, and optionally coated with a soluble protective coating and admixed with the calcium granules coated with the delayed release material. Finally, hard gelatin capsules are filled with the coated granules.

For the tablet form, a core of the calcium source, plus any optional ingredients, is formed. This core is then surrounded by the coating of delayed release material. The iron source is then adhered to this coated core. To prevent the oxidation of the iron source, a protective layer of a suitable water-soluble or acid-soluble material such as hydroxypropyl methyl cellulose or Eudragit E sold by Rohm Pharma can be coated onto the iron source.

In making the tablet form of the supplement, the calcium source, plus any optional ingredients such as binders (e.g. gelatin, polyvinyl pyrrolidone), lubricants (e.g. stearates), and disintegrants (e.g., starch, sodium starch glycolate, cellulosics) are either directly compacted to form a core or else granulated to form a free-flowing powder which is then compacted to form the core. The coating of delayed release material is then applied to the core using a fluidized bed, pan coater, or compression coating methods. Optionally, granules of the calcium source can be made, coated with the delayed release material, and then the coated granules compressed to form the coated core. The iron source is then applied by spraying, dipping or coating it onto the coated core. Finally, the iron source can be optionally surrounded with the protective coating layer by spraying, dipping or coating.

C.   Concurrent Administration of Dietary Calcium and Iron.

The present invention also relates to a method for supplying both dietary calcium and iron to mammals and particularly

0208362

humans. This method comprises the steps of orally administering to the mammal, in a concurrent fashion: (a) a calcium supplement comprising a nutritionally effective amount of a source of absorbable and bioavailable calcium surrounded by a delayed release coating as previously defined; and (b) an iron supplement comprising a nutritionally effective amount of a source of absorbable and bioavailable iron. The iron source is positioned so that the delayed release coating substantially prevents the calcium source from inhibiting the biological adsorption of the iron in the iron source.

In practicing this method, the individual can take one of the previously defined dietary calcium and iron supplements. However, the method of the present invention can also be practiced by concurrently taking a calcium supplement comprising a source of absorbable and bioavailable calcium which is surrounded by a delayed release coating and a separate iron supplement containing a source of absorbable and bioavailable iron. As used herein, the term "concurrent administration" refers to the taking of the calcium source and iron source simultaneously or substantially simultaneously, e.g., within 15 minutes of each other.

## Specific Embodiments of Dietary Calcium and Iron Supplements

### A. Capsule Form

A slurry of calcium carbonate and povidone (K-90 grade) was prepared according to the following formula:

| | |
|---|---|
| Calcium Carbonate | 33.6% |
| Povidone | 2.5% |
| Water | 63.9% |
| | 100.0% |

The above slurry (4500 g) was coated onto 500 g of 25/30 mesh sugar beads using a Wurster fluidized bed coater. The operating conditions were as follows:

| Inlet Air Temp. | 90°C |
| Slurry Feed Rate | 21 g/min |
| Atomizing Air Pressure | 2 bar |
| Inlet Air Flow Adjusted to Maintain Outlet Air Temp of | 30-35°C |

This provided approximately a 3:1 calcium carbonate/povidone coating on the sugar beads.

An enteric coating solution was prepared according to the following formula:

| Cellulose Acetate Phthalate | 12.0% |
| Castor Oil | 3.0% |
| Acetone | 82.4% |
| Water | 2.6% |
| | 100.0% |

The above solution (373 g) was coated onto 500 g of the calcium carbonate/povidone coated sugar beads (hereafter calcium carbonate substrate) using a Wurster fluidized bed coater. The target operating conditions were as follows:

| Inlet Air Temp. | 50°C |
| Solution Feed Rate | 92 ml/min |
| Atomizing Air Pressure | 2.4 bar |
| Inlet Air Flow Adjusted to Maintain Outlet Air Temp of | 30-35°C |

This provided a 10% w/w coating of cellulose acetate phthalate/castor oil on the calcium carbonate substrate.

An iron sulfate and zinc sulfate slurry was prepared according to the following formula:

- 15 -

| Iron Sulfate | 11.7% |
| Zinc Sulfate | 12.7% |
| Povidone | 1.9% |
| Tween 80 | 0.5% |
| Water | 73.2% |
| | 100.0% |

0208362

This slurry (50 g) was applied to 500 g of the cellulose acetate phthalate coated calcium carbonate substrate using a Wurster fluidized bed coater. The target operating conditions were as follows:

| Inlet Air Temp. | 90°C |
| Slurry Feed Rate | 5 g/min |
| Atomizing Air Pressure | 2 bar |
| Inlet Air Flow Adjusted to Maintain Exit Air Temp of | 30-35°C |

This provided granules having approximately a 60:1 calcium to iron weight ratio. Hard gelatin capsules were then filled with the finished granules.

The finished granules can be optionally coated with a protective film such as hydroxypropyl methylcellulose. This film can be applied from a 5% solution in ethanol using a Wurster fluidized bed coater. The operating conditions are similar to those employed for coating the cellulose acetate phthalate.

B.  Tablet Form

Destab calcium carbonate (90/10 calcium carbonate/gum acacia, Desmol Chemical Corporation, St. Louis, MO, 694 mg/tablet), Frodex 22 (dextrose and hydrolyzed starch, 150 mg/tablet), and sodium starch glycolate (50 mg/tablet) were added to a V-blender through a 30 mesh screen and mixed about 15 min. Magnesium stearate (2 mg/tablet) was then added

- 16 -

through a 60 mesh screen and the total mixture mixed for an additional 5 min. The mixture was then compressed into tablets using 7/16 inch standard concave punches on a rotary tablet press. Sufficient pressure was used to produce tablets with hardness greater than 5 kg.

An enteric coating solution was prepared according to the following formula:

| | |
|---|---|
| Cellulose Acetate Phthalate | 5.5% |
| Castor Oil | 1.4% |
| Acetone | 92.2% |
| Water | 0.9% |
| | 100.0% |

The above solution (2170 g) was applied to 2.63 kg of tablets using a Hi-Coater type of pan coater. The target operating conditions were as follows:

| | |
|---|---|
| Inlet Air Temperature | $40^{\circ}C$ |
| Coating Solution Flow Rate | 70 ml/min |
| Pattern Air | 40 CLPM |
| Atomizing Air | 40 CLPM |
| Rotation Speed | 8-15 rpm |

This provided an approximately 5.5% coating of cellulose acetate phthalate/castor oil on the tablets.

An iron sulfate and zinc sulfate coating slurry is prepared as in the first example. This slurry (400 g) is applied to the cellulose acetate phthalate coated tablets using the Hi-Coater. The target operating conditions are as follows:

| | |
|---|---|
| Inlet Air | $90^{\circ}C$ |
| Coating Solution Flow Rate | 50 ml/min |
| Pattern Air | 40 CLPM |
| Atomizing Air | 40 CLPM |
| Rotation Speed | 8-15 rpm |

This provides tablets having approximately a 50:1 calcium to iron weight ratio. The tablets can be optionally coated with a film of hydroxypropyl methyl cellulose to prevent oxidation of the iron.

## Iron Absorption Studies Involving
## Coated and Noncoated Calcium Source

The effect of enterically coated versus noncoated calcium sources on iron absorption was studied in rats. Four groups of rats (N =7) were used in the studies. One group of rats was orally dosed with water and ferrous sulfate. The second group was orally dosed with solid (powdered) calcium carbonate and ferrous sulfate (calcium to iron weight ratio of 120:1). The third group was orally dosed with an aqueous suspension of calcium carbonate and ferrous sulfate (calcium to iron weight ratio of 120:1). The fourth group was orally dosed with an aqueous suspension of ferrous sulfate and enterically coated calcium carbonate (powdered).

Each of the dosage forms contained radiolabeled $^{59}$Fe. Iron absorption was determined by measuring whole body retention of the radiolabeled $^{59}$Fe. The results were as follows:

| Treatment | % Iron Retention | Index |
|---|---|---|
| Water | $33.2 \pm 3.7$ | 100 |
| $CaCO_3$ (solid) | $21.6 \pm 1.9$ | 65 |
| $CaCO_3$ (suspension) | $20.1 \pm 3.3$ | 61 |
| $CaCO_3$ (enterically coated) | $42.1 \pm 3.4$ | 127 |

As can be seen, the uncoated calcium carbonate (solid or suspension) significantly decreased iron absorption. By contrast, the enterically coated calcium carbonate did not decrease iron absorption.

CLAIMS

1. A dietary calcium and iron supplement, which comprises:

(1) a nutritionally effective amount of a source of absorbable and bioavailable calcium;

(2) a delayed release coating surrounding said calcium source;

(3) a nutritionally effective amount of a source of absorbable and bioavailable iron;

said iron source being positioned in the supplement so that said delayed release coating substantially prevents said calcium source from inhibiting the biological absorption of iron in said iron source.

2. The supplement of Claim 1 wherein said calcium source comprises a calcium salt selected from the group consisting of calcium carbonate, calcium bicarbonate, calcium citrate, calcium malate, calcium phosphate, calcium hydrogen phosphate, calcium dihydrogen phosphate, calcium lactate, calcium orotate, calcium oxide, calcium sulfate, calcium gluconate, calcium hydroxyapatite, calcium levulinate, calcium caseinate, calcium glubionate and mixtures thereof.

3. The supplement of Claim 2 wherein said calcium salt is selected from the group consisting of calcium carbonate and mixtures of calcium citrate and calcium malate.

4. The supplement of Claim 1 wherein said iron source comprises an iron salt selected from the group consisting of ferrous sulfate, ferrous chloride, ferrous fumarate, ferrous gluconate, ferrous ascorbate, ferrous succinate, ferrous lactate, ferric ammonium citrate, ferric pyrophosphate, ferroglycine sulfate and mixtures thereof.

5. The supplement of Claim 4 wherein said iron salt is selected from the group consisting of ferrous sulfate and ferrous fumarate.

6. The supplement of Claim 1 wherein said delayed release coating is selected from the group consisting of enteric coatings, enteral enzyme degradable coatings, infusive coatings and slowly soluble coatings.

7. The supplement of Claim 6 wherein said delayed release coating is an enteric coating.

8. The supplement of Claim 7 wherein said enteric coating comprises a material selected from the group consisting of hydroxypropyl methylcellulose phthalate, cellulose acetate phthalate, acrylic anionic polymers and polyvinyl acetyl phthalate.

9. The supplement of Claim 8 wherein said material is cellulose acetate phthalate.

10. The supplement of Claim 1, wherein the weight ratio of calcium to iron is from about 10:1 to about 150:1.

11. The supplement of Claim 10 wherein said weight ratio is from about 20:1 to about 90:1.


12. A dietary calcium and iron supplement capsule, which comprises:

   (1) of a source of absorbable and bioavailable calcium in an amount of from about 100 to about 1000 mg. by weight calcium, said calcium source being in the form of a multiplicity of granules;

   (2) a delayed release coating surrounding each of said granules;

   (3) a source of absorbable and bioavailable iron in an amount of from about 6 to 25 mg. by weight iron, said iron source being adhered to the exterior surface of said delayed release coating of each of said granules;

(4)   the weight ratio of calcium to iron being being from about 20:1 to about 90:1; and

(5)   an edible casing which contains said granules.

13.   The capsule of Claim 12, wherein said granules have a particle size of from about 250 to about 2000 microns.

14.   The capsule of Claim 13 wherein said calcium source comprises a calcium salt selected from the group consisting of calcium carbonate and mixtures of calcium citrate and calcium malate.

15.   The capsule of Claim 14 wherein said iron source comprises an iron salt selected from the group consisting of ferrous sulfate and ferrous fumarate.

16.   The capsule of Claim 15 wherein said delayed release coating is an enteric coating.

17.   A dietary calcium and iron supplement tablet, which comprises:

(1)   a core comprising a source of absorbable and bioavailable calcium in an amount of from about 100 to about 1000 mg. by weight calcium;

(2)   a delayed release coating surrounding said core;

(3)   a source of absorbable and bioavailable iron adhered to the exterior surface of said delayed release coating in an amount of from about 6 to about 25 mg. by weight iron;

(4)   the weight ratio of calcium to iron being from about 30:1 to about 90:1; and

(5)   a water-soluble or acid-soluble protective coating surrounding said iron source.

18. The tablet of Claim 17 wherein said calcium source comprises a calcium salt selected from the group consisting of calcium carbonate and mixtures of calcium citrate and calcium malate.

19. The tablet of Claim 18 wherein said iron source comprises an iron salt selected from the group consisting of ferrous sulfate and ferrous fumarate.

20. The tablet of Claim 19 wherein said delayed release coating is an enteric coating.

21. A method for supplying dietary calcium and iron to mammals, which comprises the steps of orally administering to a mammal, in a concurrent fashion: (a) a calcium supplement comprising a nutritionally effective amount of a source of absorbable and bioavailable calcium which is surrounded by a delayed release coating; and (b) an iron supplement comprising a nutritionally effective amount of a source of absorbable and bioavailable iron, the iron source being positioned so that the delayed release coating substantially prevents said calcium source from inhibiting the biological absorption of iron in the iron source.

22. The method of Claim 21 wherein the calcium source comprises a calcium salt selected from the group consisting of calcium carbonate and mixtures of calcium citrate and calcium malate.

23. The method of Claim 22 wherein the iron source comprises an iron salt selected from the group consisting of ferrous sulfate and ferrous fumarate.

24. The method of Claim 23 wherein the delayed release coating is an enteric coating.

25. The method of Claim 24, wherein the weight ratio of calcium to iron is from about 20:1 to about 150:1.

0208362

26. The method of Claim 25 wherein said weight ratio is from about 30:1 to about 90:1.

27. The method of Claim 21 wherein said calcium source and iron source are orally administered in the same supplement.

TAP2:dr 6080

## DOCUMENTS CONSIDERED TO BE RELEVANT

| Category | Citation of document with indication, where appropriate, of relevant passages | Relevant to claim | CLASSIFICATION OF THE APPLICATION (Int. Cl 4) |
|---|---|---|---|
| D,A | US-A-2 410 417 (C.N. ANDERSEN) <br><br> * claim 1; column 5, lines 13-45; column 9, lines 45-66 * | 1,2,4, 6-8 | A 61 K 33/06 <br> A 61 K 33/26 <br> A 61 K 9/52 <br> A 61 K 9/24 |
| D,A | US-A-4 431 634 (L. ELLENBOGEN) <br> * claim 9 * | 1,2,4 | |
| A | US-A-3 279 997 (H.D. SCHNEYER) <br><br> * claim 1; figure * | 1,2,6, 7 | |
| D,A | US-A-3 345 265 (M.G. GRODBERG et al.) <br> * claims 1, 8 * | 1-3 | |
| A | DE-A-2 224 256 (SANOL-ARZNEIMITTEL DR. SCHWARZ GMBH) <br> * claims; example 1 * | 1,4,6, 7 | **TECHNICAL FIELDS SEARCHED (Int Cl 4)** <br><br> A 61 K 9/00 <br> A 61 K 33/00 |
| A | US-A-3 078 216 (M. GREIF) <br> * claims 1, 10, 12 * | 1,6,7 | |
| D,A | US-A-2 887 436 (O. KLIOZE et al.) <br> * claim 1; column 7, lines 4-47 * | 1 | |

--- -/-

The present search report has been drawn up for all claims

| Place of search | Date of completion of the search | Examiner |
|---|---|---|
| BERLIN | 24-09-1986 | HASS C V F |

0208362

European Patent
Office

EUROPEAN SEARCH REPORT

Application number

EP 86 20 1053

## DOCUMENTS CONSIDERED TO BE RELEVANT

Page 2

| Category | Citation of document with indication, where appropriate, of relevant passages | Relevant to claim | CLASSIFICATION OF THE APPLICATION (Int Cl 4) |
|---|---|---|---|
| A | EP-A-0 013 131 (MUNDIPHARMA AG) * claim 1 * | 1 | |
| | --- | | |
| D,A | EP-A-0 094 123 (PROCTER & GAMBLE CO.) * claims 1, 4 * | 6-8 | |
| | ----- | | |

TECHNICAL FIELDS
SEARCHED (Int. Cl 4)

The present search report has been drawn up for all claims

| Place of search | Date of completion of the search | Examiner |
|---|---|---|
| BERLIN | 24-09-1986 | HASS C V F |